Europäisches Patentamt

European Patent Office

Office européen des brevets

(19)

(11) Veröffentlichungsnummer: **0 226 985**
**A2**

# (12) EUROPÄISCHE PATENTANMELDUNG

(21) Anmeldenummer: 86117383.9

(22) Anmeldetag: 13.12.86

(51) Int. Cl.⁴: **G 01 N 33/74**
**G 01 N 33/577, C 12 P 21/00**
**C 12 N 5/00, C 07 K 15/00**

(30) Priorität: 20.12.85 DE 3545252

(43) Veröffentlichungstag der Anmeldung:
01.07.87 Patentblatt 87/27

(84) Benannte Vertragsstaaten:
AT BE CH DE ES FR GB GR IT LI LU NL SE

(71) Anmelder: Boehringer Mannheim GmbH
Sandhofer Strasse 116
D-6800 Mannheim 31(DE)

(72) Erfinder: Hübner-Parajsz, Christa, Dr.rer.nat.
Marienstrasse 11
D-8132 Tutzing(DE)

(72) Erfinder: Essig, Ulrich, Dr.
Waldpromenade 28B
D-8035 Gauting(DE)

(72) Erfinder: Herrmann, Rupert, Dr.rer.nat.
In der Au 23
D-8120 Weilheim(DE)

(72) Erfinder: Klein, Christian, Dr.
Blütenstrasse 16
D-8120 Weilheim(DE)

(54) **Verfahren und Reagenz zur Bestimmung von Testosteron sowie hierzu geeignete monoklonale Antikörper.**

(57) Gegenstand der Erfindung ist ein immunologisches Verfahren zur Bestimmung von Testosteron, wobei mindestens ein monoklonaler Antikörper verwendet wird, der gegen Testosteron gerichtet ist und mit anderen strukturell ähnlichen Steroid-Hormonon nicht oder nur wenig kreuzreagiert, sowie ein hierfür geeignetes Reagenz. Beansprucht werden ferner monoklonale Antikörper gegen Testosteron mit keiner oder nur geringer Kreuzreaktivität mit anderen Steroid-Hormonen, Verfahren zur Gewinnung dieser Antikörper sowie Hybridoma-Zellinien, die solche Antikörper produzieren.

Croydon Printing Company Ltd

Verfahren und Reagenz zur Bestimmung von Testosteron sowie
hierzu geeignete monoklonale Antikörper

Die Bestimmung von Testosteron gewinnt in der Diagnostik zunehmende Bedeutung. Beim Mann wird Testosteron bestimmt, um Störungen der inkretorischen Hodenfunktion zu diagnostizieren. Bei
der Frau wird eine Testosteron-Bestimmung durchgeführt, wenn
Symptome vorliegen, die für eine verstärkte ovarielle oder
adrenale Androgenbildung sprechen.

Testosteron ist das wichtigste Androgen. Strukturelles Merkmal
aller Androgene ist das C19-Steroidgerüst. Die Bildung von Testosteron im menschlichen Organismus wird durch die Hypophyse
reguliert. Das hypophysäre Gonadotropin LH (luteinisierendes
Hormon) stimuliert in den Leydigzellen die Synthese des Testosterons, das wiederum die Gonadotropin-Sekretion über einen negativen Feedback-Mechanismus reguliert. Pro Tag werden beim
Mann ca. 7,2 mg und bei der Frau ca. 0,2 mg Testosteron gebildet.

Die Funktion des Testosterons besteht in der Ausbildung der
männlichen Geschlechtsorgane während der prä- und postnatalen
Entwicklung und der sekundären Geschlechtsmerkmale in der Pubertät. Im erwachsenen männlichen Organismus ist die kontinuierlich erfolgende Produktion des Hormons für die Reifung der
Spermien und vor allem für die Tätigkeit der akzessorischen
Drüsen des Genitaltraktes (Prostata und Samenblase) wichtig.
Auf den Stoffwechsel hat Testosteron eine anabole Wirkung. Am
Wirkungsort wird Testosteron zum $5\alpha$-Dihydro-Testosteron (DHT)
reduziert. Mit seiner fünffach höheren Aktivität kann dieses
zumindest für die Prostata als die eigentliche Wirkform angesehen werden.

Die physiologische Konzentration von Testosteron im Plasma liegt üblicherweise in den folgenden Bereichen (in ng/ml Plasma):

| | |
|---|---|
| <u>Männer</u> vor der Pubertät | 0,2 - 0,8 |
| Erwachsene < 40 Jahre | 4 - 12 |
| Erwachsene > 40 Jahre | 3 - 8 |
| <u>Frauen</u> vor der Pubertät | 0,1 - 0,3 |
| Erwachsene | 0,2 - 0,8 |

Erniedrigte Plasmawerte werden beim Mann beispielsweise gefunden bei androgenen Ausfallerscheinungen (z. B. Verminderung der Bartbehaarung, Prostataadrophie usw.), Hodenadrophie und Hypoplasie, Hinweis auf einen Prozeß im Hypophysen-Hypothalamus-Bereich, Klimakterium virile, Verlust von Libido und Potentia coeundi, Impotentia generandi, Pubertas tarda, Wachstumsstörungen, Kryptorchismus, kongenitales Syndrom mit Hodenminderfunktion, Klinefelter-Syndrom. Bei der Pubertas praecox werden erhöhte Testosteron-Werte gemessen, z. B. aufgrund eines seltenen Leydig-Zelltumors. Zur Differenzierung von primärem Hypogonadismus (Hodenfunktionsstörungen aufgrund eines organischen Schadens) und sekundärem Hypogonadismus (ungenügende Funktion der Hypophyse) wird bei erniedrigten Testosteron-Werten zusätzlich eine LH-Bestimmung durchgeführt.

Erhöhte Testosteron-Werte werden bei der Frau gemessen bei Hirsutismus, adrenogenitalem Syndrom, Stein-Leventhal-Syndrom, androgenbildenden Tumoren des Ovars oder der Nebennierenrinde, Nebennierenrinden-Hyperplasie, wie z. B. beim Cushing-Syndrom. Auch nach einer Östrogen-Behandlung, während der Schwangerschaft und bei gesteigerter Schilddrüsenfunktion sind die Testosteron-Konzentrationen erhöht.

Bei Alkoholabusus, Streß, schweren Operationen und bei gewissen Arzneimitteln liegt eine erniedrigte Testosteron-Konzentration im Plasma vor.

Für die Bestimmung von Testosteron eignen sich vor allem immunologische Testverfahren, bei denen das Testosteron als Antigen mit einem gegen dieses Antigen gerichteten Antikörper bestimmt wird. Für solche immunologischen Bestimmungsverfahren sind Antikörper erforderlich, die möglichst spezifisch gegen Testosteron gerichtet sind. Insbesondere die Testosteron-Bestimmung im Serum der Frau macht den Einsatz eines hochspezifischen Antiserums erforderlich, da hier Verbindungen des Androgen-Stoffwechsels in Konzentration vorliegen, die derjenigen des Testosterons vergleichbar sind bzw. sie sogar übertreffen. Beim Mann sind die Konzentrationsverhältnisse im Hinblick auf die Testosteron-Bestimmung etwas günstiger. Hier sind die Verbindungen des androgenen Stoffwechsels in einer Konzentration vertreten, die ca. eine Größenordnung unter derjenigen des Testosterons liegen.

Die Gewinnung von Antikörpern mit hinreichend hoher Spezifität gegen Testosteron ist jedoch mit erheblichen Schwierigkeiten verbunden, da wegen der geringen strukturellen Unterschiede zwischen verwandten Steroiden ziemlich hohe Kreuzreaktivitäten mit anderen Steroiden zu beobachten ist. Es ist bekannt, daß insbesondere wegen der geringen strukturellen Unterschiede zwischen Testosteron und dem Abbauprodukt DHT die Herstellung eines für Testosteron spezifischen Antiserums mit niedriger Kreuzreaktion zu DHT die größten Probleme bereitet (vergleiche K. Lübke u. a. in: Immunologische Teste für niedermolekulare Wirkstoffe, Georg Thieme Verlag Stuttgart 1978, Kapitel 3.6.2., Seite 101-103). Die hohen Anforderungen an die Spezifität sollten am ehesten von monoklonalen Antikörpern zu erfüllen sein.

Monoklonale Antikörper gegen Testosteron sind bereits bekannt (vergleiche J. Endocr. 100 (1984), Seite 367 - 376; J. Steroid. Biochem. 19 (1983), Seite 1605 - 1610 sowie J. Steroid. Biochem. 22 (1985), Seite 169 - 175). Diese Antikörper werden in bekannter Weise gewonnen. Als Immunogene werden dabei Konjugate aus Testosteron und einem immunogenen Trägermaterial gewonnen, wobei die Verknüpfung zwischen Testosteron und dem immunogenen Trägermaterial über die 3-, 15- oder 17-Stellung erfolgt. Alle diese vorbekannten monoklonalen Antikörper gegen Testosteron zeigen jedoch eine sehr starke Kreuzreaktivität mit strukturell nahe verwandten Steroidhormonen und sind somit für eine selektive Bestimmung von Testosteron nicht geeignet.

Aufgabe der Erfindung war es, ein neues immunologisches Verfahren und Reagenz bereit zu stellen, mit dessen Hilfe Testosteron auch in Gegenwart von anderen Steroid-Hormonen spezifisch erfaßt werden kann. Gelöst wird diese Aufgabe durch das erfindungsgemäße immunologische Verfahren und Reagenz.

Gegenstand der Erfindung ist daher ein immunologisches Verfahren zur Bestimmung von Testosteron, bei dem mindestens ein monoklonaler Antikörper eingesetzt wird, der spezifisch gegen Testosteron gerichtet ist und mit anderen Steroid-Hormonen 5 % oder weniger kreuzreagiert, sowie ein hierfür geeignetes Reagenz.

Als immunologische Bestimmungmethode eignen sich im Prinzip alle gängigen Immuno-Assays, wie Radio-Immuno-Assay, Enzym-Immuno-Assay, Fluoreszenz-Immuno-Assay usw. Ferner sind sämtliche Verfahrensvarianten, wie kompetitiver Immuno-Assay, JEMA-Verfahren usw., anwendbar. Zur Markierung eignen sich die für die jeweilige Bestimmungsmethode üblichen Mittel. So werden bei einem Radio-Immuno-Assay Radioisotope, beispielsweise $^{125}$J, zur Markierung verwendet. Für einen Enzym-Immuno-Assay sind sämtliche, hierfür üblicherweise eingesetzten Enzyme, beispielsweise Peroxidase oder ß-Galactosidase, geeignet. Für einen Fluoreszenz-Immuno-Assay sind die üblichen fluoreszierenden Gruppen als Marker brauchbar. Einzelheiten dieser verschiedenen Testmethoden und Verfahrensvarianten sind dem Fachmann bekannt.

Zur Bestimmung von Testosteron hat sich als besonders zweckmäßig ein kompetitiver Enzym-Immuno-Assay oder ein Verfahren nach
dem JEMA-Prinzip erwiesen. Bei dem kompetitiven Enzym-Immuno--
Assay konkurriert das zu bestimmende Testosteron mit einer bekannten Menge markierten Testosteron um die Bindungsstellen des
monoklonalen Antikörpers. Das Testverfahren kann auch so durchgeführt werden, daß das zu bestimmende Testosteron und trägergebundenes Testosteron um im Unterschuß vorhandene Bindungsplätze des markierten monoklonalen Antikörpers konkurrieren.
Der an das trägerfixierte Testosteron gebundene Anteil des markierten monoklonalen Antikörpers wird anhand der Markierung bestimmt. Diese Variante kann auch so abgewandelt werden, daß der
monoklonale Antikörper nicht markiert eingesetzt wird. Der an
das trägerfixierte Testosteron gebundene Antikörperanteil wird
dann ermittelt, indem mit einem gegen den Fc-Teil des Antikörpers gerichteten markierten Antikörper inkubiert und der gebundene markierte Anteil bestimmt wird. Bei dem JEMA-Verfahren
wird markierter, monoklonaler Antikörper im Überschuß zugegeben. Der überschüssige, nicht an Testosteron gebundene markierte Antikörper wird mit Hilfe einer Hapten-Trägermatrix aus der
Lösung entfernt. Die verschiedenen Varianten dieser Testmethoden sowie Einzelheiten zur Durchführung dieser Verfahren sind
in der Literatur ausführlich beschrieben.

Das Wesentliche der vorliegenden Erfindung ist darin zu sehen,
daß es überraschenderweise gelang, für diese immunologischen
Verfahren monoklonale Antikörper bereitzustellen, die spezifisch gegen Testosteron gerichtet sind, und die daher eine spezifische Bestimmung von Testosteron ermöglichen.

Ein weiterer Gegenstand der Erfindung sind daher monoklonale
Antikörper gegen Testosteron, deren Kreuzreaktivität mit anderen Steroid-Hormonen 5 % oder weniger beträgt.

- 6 -                                      0226985

Zur Gewinnung der erfindungsgemäßen monoklonalen Antikörper
wird Testosteron zunächst mit einem immunogenen Trägermaterial
verknüpft. Als immunogene Trägermaterialien eignen sich alle
üblicherweise für diesen Zweck verwendeten Stoffe, beispielsweise Albumine, wie Rinderserum-Albumin, Edestin usw. Die Verknüpfung von Testosteron mit dem Trägermaterial erfolgt nach an
sich bekannten Methoden. Anschließend werden Versuchstiere,
beispielsweise Mäuse, mit dem immunogenen Konjugat immunisiert.
Zur Immunisierung wird das Immunogen beispielsweise in Kombination mit einem Adjuvans in üblicher Weise verabreicht. Bevorzugt wird als Adjuvans Aluminiumhydroxid zusammen mit Bordetella pertussis oder Freundschem Adjuvans eingesetzt. Die Immunisierung erfolgt vorzugsweise über mehrere Monate mit mindestens
vier Immunisierungen in vier- bis sechswöchigem Abstand (Injektion intraperitoneal).

Aus so immunisierten Tieren werden B-Lymphozyten gewonnen, die
mit einer permanenten Myelomzellinie fusioniert werden. Die Fusionierung erfolgt nach dem bekannten Verfahren von Köhler und
Milstein (Nature 256, 1975, S. 495 - 497). Die hierbei gebildeten Primärkulturen von Hybridzellen werden in üblicher Weise,
z. B. unter Verwendung eines handelsüblichen Zellsorters oder
durch "limiting dilution", kloniert. Es werden jeweils die Kulturen weiterverarbeitet, die in einem geeigneten Testverfahren,
beispielsweise einem Enzym-Immuno-Assay (ELISA-Verfahren), positiv gegen Testosteron und negativ bzw. nur wenig mit den anderen Steroid-Hormonen reagieren. Man erhält so mehrere Hybridoma-Zellinien, die die erfindungsgemäßen monoklonalen Antikörper produzieren. Nach bekannten Methoden können diese Zellinien
kultiviert und die von ihnen produzierten monoklonalen Antikörper isoliert werden.

Beispielhaft für auf diese Weise gewonnene Zellinien seien angeführt:

    Klon  97      (ECACC 85 12 1702) und
    Klon 160      (ECACC 85 12 1701)

Die Zellinien sind unter der jeweils angegebenen Nummer bei der Hinterlegungsstelle ECACC (European Collection of Animal Cell Cultures) hinterlegt.

Die so gewonnenen monoklonalen Antikörper zeichnen sich dadurch aus, daß sie eine sehr hohe Affinität (Affinitätskonstante in der Größenordnung von $10^{-9}$ bis $10^{-10}$ 1/mol) gegen Testosteron besitzen und mit anderen strukturell ähnlichen Steroid-Hormonen 5 % oder weniger kreuzreagieren. Besonders bevorzugt sind monoklonale Antikörper, die gegenüber 5$\alpha$-Dihydrotestosteron eine Kreuzreaktivität von 5 % oder weniger und gegenüber Androstendion eine Kreuzreaktion von 3 % oder weniger aufweisen. Zur Bestimmung der Affinität und der Kreuzreaktivität mit anderen Hormonen stehen die üblichen, dem Fachmann bekannten Verfahren zur Verfügung.

Die erfindungsgemäßen monoklonalen Antikörper eignen sich hervorragend dazu, in einer Probe, beispielsweise Serum oder Plasma, das Hormon Testosteron in Gegenwart anderer Steroid-Hormone spezifisch zu bestimmen. Für diese Bestimmungsverfahren können die monoklonalen Antikörper als solche oder Fragmente hiervon, welche die entsprechenden immunologischen Eigenschaften aufweisen, beispielsweise Fab-Fragmente, verwendet werden. Unter dem Begriff "monoklonale Antikörper" werden daher sowohl die vollständigen Antikörper als auch die Fragmente verstanden.

Die Erfindung wird durch die folgenden Beispiele näher erläutert:

Beispiel 1

## Gewinnung von monoklonalen Antikörpern gegen Testosteron

## Immunisierung von Mäusen mit Testosteron-Konjugat

Balb/c-Mäuse, 8-12 Wochen alt, wurden mit 100 µg Testosteron-3-carboxymethyloxim-Edestin (hergestellt nach J. Biol. Chem. 222 (1957), Seite 713-727) in komplettem Freundschen Adjuvans intraperitoneal erstimmunisiert. Nach sechs Wochen wurden drei weitere Immunisierungen in vierwöchigen Abständen durchgeführt. Dabei wurden 100 µg Testosteron-3-carboxymethyloxim-Edestin plus $10^9$ Keime Bortedella pertussis, adsorbiert an Aluminiumhydroxid intraperitoneal verabrecht. 4 Tage und 3 Tage vor der Fusion wurde noch einmal mit 100 bzw. 80 µg Testosteron-3-carboxymethyloxim-Edestin i.p. bzw. i.v. immunisiert.

## Fusion

Milzzellen einer immunisierten Maus wurden mit P3x63Ag8-653 Myelomzellen (ATCC-CRL 8375) im Verhältnis 1:5 gemischt und zentrifugiert (10 Minuten, 300 g, 4° C). Die Zellen wurden noch einmal mit BSS (Balanced Salt Solution)-Puffer gewaschen und bei 400 g in einem 50 ml Spitzröhrchen zentrifugiert. Der Überstand wurde abgekippt, das Zellsediment aufgelockert, 1 ml PEG (MG 4000, Merck) dazugegeben und durchpipettiert. Nach 1 Minute im Wasserbad wurden bei Raumtemperatur 5 ml RPMI 1640-Medium (RPMI = Rosewell Parker Memory Institut) ohne FKS (fötales Kälberserum) in einem Zeitraum von 4 bis 5 Minuten zugetropft, durchmischt, auf 50 ml mit Medium aufgefüllt und anschließend 10 Minuten bei 400 g, 4° C zentrifugiert. Die sedimentierten Zellen wurden in RPMI 1640-Medium + 10 % FKS aufgenommen und je $5 \times 10^4$ - $1 \times 10^5$ Milzzellen oder $5 \times 10^4$ Peritoneal-Exudat-Zellen als "Futterzellen" zugefügt. Am nächsten Tag wurde Hypoxanthin-Azaserin-Selektionsmedium (100 mM Hypoxanthin, 1 µg/ml Azaserin) zugegeben.

Circa 7 - 10 Tage nach der Fusion waren bereits viele Klone sichtbar. Der Überstand der Primärkulturen würde nach einem in Beispiel 2 beschriebenen ELISA-Verfahren getestet. Primärkulturen, die nur eine geringe Kreuzreaktion mit anderen Steroiden zeigten, wurden mittels FACS (Fluorescence activated cell sorter) in 96 -well-Zellkulturplatten kloniert. Als "Futterzellen" wurden $1 \times 10^4$ Peritoneal-Exudat-Zellen oder $2 \times 10^4$ Milzzellen pro "well" zugegeben. Auf diese Weise konnten beispielsweise die beiden Hybridoma-Zellinien

ECACC 85 12 1702 (= Klon 97) und
ECACC 85 12 1701 (= Klon 160)

isoliert werden, die bei der Hinterlegungsstelle ECACC unter der jeweils angegebenen Hinterlegungsnummer hinterlegt worden sind.

Induktion von Ascites

$5 \times 10^6$ Hybridzellen wurden i.p. in 1-2x mit 0,5 ml Pristan vorbehandelte Mäuse gespritzt. Ascites mit einer IgG-Konzentration von 5-20 mg/ml konnte 1-3 Wochen danach gewonnen werden. Hieraus können in üblicher Weise die Antikörper isoliert werden. Diese monoklonalen Antikörper sind spezifisch gegen Testosteron gerichtet und zeigen nur eine geringe Kreuzreaktivität mit anderen Steroid-Hormonen. Die monoklonalen Antikörper werden mit MAK 97 (von Klon 97) bzw. MAK 160 (von Klon 160) bezeichnet.

## Beispiel 2

## Screening-Test auf Antikörper gegen Testosteron

Um die Anwesenheit und Spezifität von Antikörpern gegen Testosteron im Serum immunisierter Mäuse oder in dem Kulturüberstand der Hybridzellen oder in Ascites zu erkennen, wird ein ELISA-Verfahren als Testprinzip verwendet: Mikrotiterplatten werden mit 1 µg Testosteron-3-carboxymethyloxim-RSA/ml Beschichtungspuffer (0,2 M Natriumcarbonat/-Bicarbonat, pH 9,3-9,5) bei 37° C eine Stunde lang beschichtet. Es wird 10 Minuten mit 0,9 % Natriumchloridlösung und 1 % Albuminlösung nachbehandelt. Anschließend wird mit 0,9 % Natriumchloridlösung gewaschen. Danach wird bei 37° C eine Stunde mit 100 µl Probe inkubiert und erneut mit 0,9 % Natriumchloridlösung gewaschen. Um eine mögliche Kreuzreaktion mit anderen Steroiden zu prüfen, werden der Probelösung 100 µg - 1 µg/ml des zu prüfenden Steroids zugesetzt. Eine Erniedrigung des Meßsignals in Gegenwart anderer Steroide bedeutet Kreuzreaktion. Es folgt eine weitere Inkubation (eine Stunde, 37° C) mit 450 mU/ml eines Schaf-Fabanti-- Maus-Fcγ-Peroxidase-Konjugats. Nach einem erneuten Waschschritt mit 0,9 % Natriumchlorid wird die Peroxidaseaktivität in üblicher Weise bestimmt (beispielsweise mit ABTS, 30 Minuten bei Raumtemperatur, abgelesen wird die Extinktionsdifferenz, $\Delta$ mE, bei 405 nm).

**Beispiel 3**

Bestimmung der Kreuzreaktion mit anderen Steroiden

Der Test wird, wie in Beispiel 2 beschrieben, durchgeführt.

Zu dem monoklonalen Antikörper wird jeweils das auf Kreuzreaktion zu prüfende Antigen (im besonderen $5\alpha$-Dihydrotestosteron und Androstendion) in steigender Konzentration zugegeben. Die Kreuzreaktion wird anschließend nach folgender Formel berechnet:

$$\% \text{ Kreuzreaktion} = \frac{C \text{ (Testosteron)}}{C \text{ (kreuzreagierendes Antigen)}} \times 100$$

C = Konzentration des Antigens, die zur Erreichung von 50 % des max. Signals erforderlich ist.

In der folgenden Tabelle sind die ermittelten Werte für die monoklonalen Antikörper MAK 97 und MAK 160 zusammengestellt.

| Bezeichnung des MAK | Kreuzreaktion in % mit | |
|---|---|---|
| | $5\alpha$-Dihydrotestosteron | Androstendion |
| 97 | 5 | 3 |
| 160 | 4 | 3 |

## Beispiel 4

## Bestimmung von Testosteron mittels Enzymimmunoassay

Mikrotiterplatten werden mit einer Lösung, die 1 $\mu$g Testosteron-3-carboxymethyloxim-RSA/ml Beschichtungspuffer (0,2 M Natriumcarbonat/-bicarbonat, pH 9,3-9,5) bei 37° C eine Stunde lang beschichtet. Es wird zehn Minuten mit 0,9 % Natriumchloridlösung und 1 % Albuminlösung nachbehandelt. Anschließend wird mit 0,9 %iger Natriumchloridlösung gewaschen. Danach wird bei 37° C eine Stunde mit 100 $\mu$l Probe inkubiert. Die Probe wird erhalten, indem die Lösung mit dem zu bestimmenden Testosteron zu gleichen Teilen mit einer 0,9 %igen Natriumchlorid-Lösung, die den erfindungsgemäßen monoklonalen Antikörper MAK 160 in einer Konzentration von 5 $\mu$g/ml enthält, 30 Minuten bei Raumtemperatur inkubiert. Es wird erneut mit 0,9 % Natriumchloridlösung gewaschen. Anschließend wird eine Stunde bei 37° C mit 450 mU/ml eines Schaf-Fab-anti-Maus-Fc$\gamma$-Peroxidase--Konjugats inkubiert.

Nach einem erneuten Waschschritt mit 0,9 % Natriumchlorid wird die Peroxidaseaktivität durch Umsetzung mit ABTS, 30 Minuten bei Raumtemperatur, und Ablesen der Extinktionsdifferenz, $\Delta$ mE, bei 405 nm bestimmt.

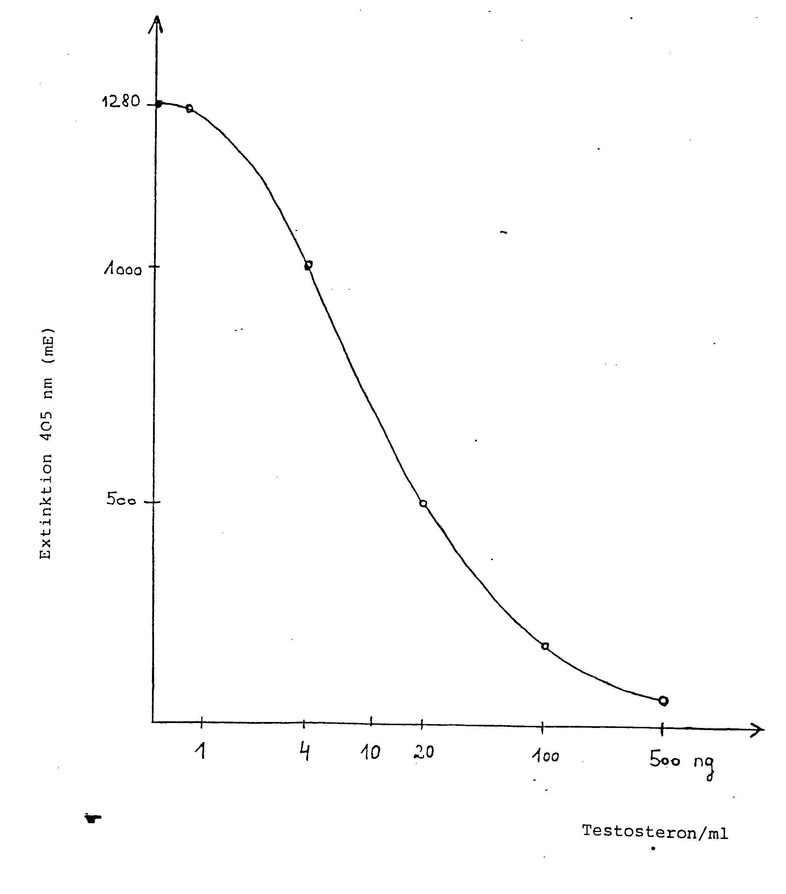

Zur Herstellung einer Eichkurve werden Lösungen eingesetzt, die Testosteron in verschiedenen, definierten Konzentrationen enthalten. Eine in vorstehender Weise gewonnene Eichkurve ist in Abbildung 1 dargestellt. Anhand dieser Eichkurve können unbekannte Testosteron-Konzentrationen ermittelt werden.

<u>Patentansprüche</u>

1. Immunologisches Verfahren zur Bestimmung von Testosteron, dadurch gekennzeichnet, daß mindestens ein monoklonaler Antikörper verwendet wird, der gegen Testosteron gerichtet ist und mit anderen Steroid-Hormonen 5 % oder weniger kreuzreagiert.

2. Verfahren gemäß Anspruch 1, dadurch gekennzeichnet, daß mindestens ein monoklonaler Antikörper verwendet wird, der gegen Testosteron gerichtet ist und mit 5$\alpha$-Dihydrotestosteron 5 % oder weniger und mit Androstendion 3 % oder weniger kreuzreagiert.

3. Reagenz zur Bestimmung von Testosteron, dadurch gekennzeichnet, daß es mindestens einen monoklonalen Antikörper enthält, der gegen Testosteron gerichtet ist und mit anderen Steroid-Hormonen 5 % oder weniger kreuzreagiert.

4. Reagenz gemäß Anspruch 3, dadurch gekennzeichnet, daß es mindestens einen monoklonalen Antikörper enthält, der gegen Testosteron gerichtet ist und mit 5$\alpha$-Dihydrotestosteron 5 % oder weniger und mit Androstendion 3 % oder weniger kreuzreagiert.

5. Monoklonaler Antikörper gegen Testosteron, dadurch gekennzeichnet, daß er mit anderen Steroid-Hormonen 5 % oder weniger kreuzreagiert.

6. Monoklonaler Antikörper gemäß Anspruch 5, dadurch gekennzeichnet, daß er mit 5$\alpha$-Dihydrotestosteron 5 % oder weniger und mit Androstendion 3 % oder weniger kreuzreagiert.

7.  Verfahren zur Gewinnung eines monoklonalen Antikörpers nach einem der Ansprüche 5 oder 6, dadurch gekennzeichnet, daß man geeignete Tiere mit einem Testosteron-Träger-Konjugat immunisiert, aus der Milz der immunisierten Tiere B-Lymphozyten gewinnt, diese mit Myelomazellen fusioniert, die gebildeten Hybridomazellen kloniert, Klone, die spezifisch gegen Testosteron gerichtete Antikörper produzieren, selektiert und die durch sie gebildeten Antikörper gewinnt.

8.  Hybridomazellinie, dadurch gekennzeichnet, daß sie monoklonale Antikörper produziert, die gegen Testosteron gerichtet sind und mit anderen Steroid-Hormonen 5 % oder weniger kreuzreagieren.

9.  Hybridomazellinie gemäß Anspruch 8, dadurch gekennzeichnet, daß sie monoklonale Antikörper produziert, die gegen Testosteron gerichtet sind und mit 5$\alpha$-Dihydrotestosteron 5 % oder weniger und mit Androstendion 3 % oder weniger kreuzreagieren.